(19) Europäisches Patentamt
European Patent Office
Office européen des brevets

(11) **EP 4 616 719 A1**

(12) **EUROPEAN PATENT APPLICATION**
published in accordance with Art. 153(4) EPC

(43) Date of publication:
**17.09.2025 Bulletin 2025/38**

(21) Application number: **23888673.3**

(22) Date of filing: **06.11.2023**

(51) International Patent Classification (IPC):
*A23J 3/14* (2006.01)     *A23J 3/22* (2006.01)
*A23J 3/34* (2006.01)     *A23L 5/00* (2016.01)
*A23L 11/00* (2025.01)    *A23L 13/00* (2016.01)
*C12N 9/48* (2006.01)     *C12N 9/54* (2006.01)
*C12N 9/62* (2006.01)     *C12P 1/00* (2006.01)
*C12P 21/06* (2006.01)

(52) Cooperative Patent Classification (CPC):
**A23J 3/14; A23J 3/22; A23J 3/34; A23L 5/00;
A23L 11/00; A23L 13/00; C12N 9/48; C12N 9/54;
C12N 9/62; C12P 1/00; C12P 21/06**

(86) International application number:
**PCT/JP2023/039955**

(87) International publication number:
**WO 2024/101326 (16.05.2024 Gazette 2024/20)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **10.11.2022 JP 2022180389**

(71) Applicants:
• **Amano Enzyme Inc.
Nagoya-shi
Aichi 460-8630 (JP)**

• **Amano Enzyme U.S.A. Co., Ltd.
Elgin, Illinois 60124 (US)**

(72) Inventors:
• **SAKAI, Kiyota
Kakamigahara-shi, Gifu 509-0109 (JP)**
• **OKUDA, Keita
Kakamigahara-shi, Gifu 509-0109 (JP)**
• **BROCHES, Nickolas
Elgin, Illinois 60124 (US)**

(74) Representative: **Müller-Boré & Partner
Patentanwälte PartG mbB
Friedenheimer Brücke 21
80639 München (DE)**

(54) **METHOD FOR PRODUCING TEXTURED VEGETABLE PROTEIN**

(57) The present invention addresses the problem of providing a technique for producing a textured plant protein that is configured such that dull color formation is suppressed. Dull color formation of the textured plant protein obtained by the present invention is suppressed by adding a nontextured plant protein material that has been treated using a glutaminase and protease to a material for texturing.

EP 4 616 719 A1

**Description**

Technical Field

The present invention relates to a method for producing a textured plant protein. More specifically, the present invention relates to a method for producing a textured plant protein in which color dullness is suppressed.

Background Art

In recent years, in the food market, the share of food using a textured plant protein material (hereinafter, also described as "substitute meat") as a substitute for animal meat has increased. Textured plant protein-containing foods were once targeted at some consumers, such as vegetarian or vegan. However, in recent years, the trend of demand has also changed, and attention has been paid again as awareness of, for instance, health consciousness, diet, environmental problems, and animal welfare has increased.

Animal meat generally exhibits a bright color and/or a vivid color tone such as red color of livestock meat, pink color of salmon, and white color of cuttlefish. On the other hand, the substitute meat generally exhibits a grayish brown color. Due to such appearance characteristics of the substitute meat, a product has been sold, to which an appearance similar to that of raw livestock meat is imparted by coloring the substitute meat with a beet liquid (NPL 1).

Meanwhile, a technology for decoloring a plant protein is also known. NPL 2 reports a method of decoloring a degreased camellia seed cake by using hydrogen peroxide. In addition, PTL 1 describes that an enzymatic hydrolysis reaction is performed under a synergistic action of an alkaline protease and an acidic protease and/or a neutral protease, and a predetermined amount of hydrogen peroxide is added to suppress generation of a colored substance.

Citation List

Patent Literature

PTL 1: CN1491572A

Non Patent Literature

NPL 1: "YES, IT'S MEAT MADE FROM PLANTS" [online], BEYOND MEAT, [searched on November 7, 2022], Internet <URL:https://www.beyondmeat.com/en-US/about/our-ingredients/>
NPL 2: International Journal of Food Properties Volume 22, 2019-Issue 1 1283-1295

Summary of Invention

Technical Problem

With the recent spread of substitute meat, it has been desired to further bring the characteristics of substitute meat closer to the characteristics of animal meat. Under such circumstances, as a method for reproducing, in the substitute meat, the appearance of the animal meat, the coloring and decoloring as described above may be a certain option. However, since coloring is performed on the grayish brown color which is an original color, the coloring has problems that vividness like raw meat cannot be reproduced, that it is necessary to impart an excessive color to conceal color dullness of the original grayish brown color, that it is impossible to reproduce animal meat of a light color such as pink, and others. In addition, in consideration of the carcinogenicity of hydrogen peroxide, the decoloring cannot meet the intention of users with high health consciousness. Therefore, in order to reproduce, in the substitute meat, the appearance of the animal meat, a new method has been sought that can inhibit the dullness of the grayish brown color exhibited by the substitute meat.

The present invention addresses the problem of providing a technology for producing a textured plant protein in which color dullness is suppressed.

Solution to Problem

The present inventors have conducted intensive research and as a result, have found that a texturing-use material can be blended with an untextured plant protein material treated with a glutaminase compound (glutaminases) and a protease to inhibit color dullness of the resulting textured plant protein. The present invention has been completed by conducting further studies based on the findings.

Specifically, the present invention provides the following items of the invention.

Item 1. A method for producing a textured plant protein, including: step 1 of treating an untextured plant protein material with a glutaminase compound (glutaminases) and a protease to prepare an enzyme-treated plant protein material; and step 2 of texturing a texturing-use material containing the enzyme-treated plant protein material.

Item 2. The method according to item 1, in which a dry weight of the enzyme-treated plant protein material is 3 parts by weight or more based on 100 parts by weight of a dry weight of the texturing-use material.

Item 3. The method according to item 1, or 2 in which the glutaminase compound is a glutaminase (EC3.5.1.2).

Item 4. The method according to any one of items 1 to 3, in which the glutaminase compound is a glutaminase compound derived from the genus Bacillus.

Item 5. The method according to any one of items 1 to 4, in which the protease is a filamentous fungus-derived protease and/or a bacterium-derived protease.

Item 6. The method according to item 5, in which the filamentous fungus protease is an acidic protease.

Item 7. The method according to item 5 or 6, in which the filamentous fungus protease is a protease derived from the genus Aspergillus.

Item 8. The method according to any one of items 5 to 7, in which the bacterium protease is a protease derived from the genus Bacillus or the genus Geobacillus.

Item 9. The method according to any one of items 1 to 8, in which the plant protein material is derived from pulses.

Item 10. The method according to any one of items 1 to 9, in which the protein material is a pea.

Item 11. A food including a textured plant protein produced by the method according to any one of items 1 to 10.

Item 12. A color dullness inhibitor for a textured plant protein, including a product obtained by enzymatic treatment of an untextured plant protein material with a glutaminase compound (glutaminases) and a protease.

Item 13. A coloring inhibitor for a textured plant protein, including a glutaminase compound (glutaminases) and a protease.

Item 14. Use of a product obtained by enzymatic treatment of an untextured plant protein material with a glutaminase compound (glutaminases) and a protease, for inhibition of color dullness in a textured plant protein.

Item 15. An application of a product obtained by enzymatic treatment of an untextured plant protein material with a glutaminase compound (glutaminases) and a protease, for inhibition of color dullness in a textured plant protein.

Item 16. A method for inhibiting color dullness of a textured plant protein, including: step 1 of treating an untextured plant protein material with a glutaminase compound (glutaminases) and a protease to prepare an enzyme-treated plant protein material; and step 2 of texturing a texturing-use material containing the enzyme-treated plant protein material.

Advantageous Effects of Invention

The present invention provides a technology for producing a textured plant protein in which color dullness is suppressed.

Brief Description of Drawings

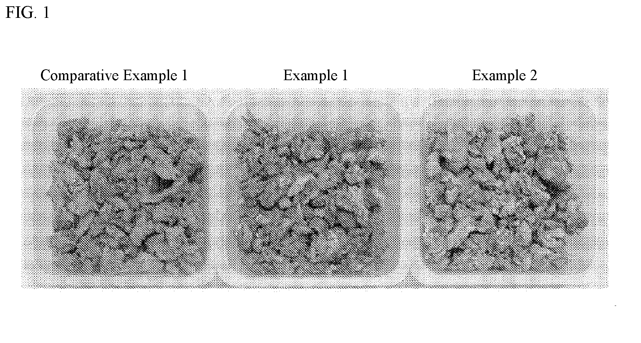

[Fig. 1] Fig. 1 shows the appearance of each textured plant protein (Examples 1 and 2) obtained by the production method of the present invention.

Description of Embodiments

1. Method for producing textured plant protein

A method for producing a textured plant protein according to the present invention is characterized by including: step 1 of treating an untextured plant protein material with a glutaminase compound (glutaminases) and a protease to prepare an enzyme-treated plant protein material; and step 2 of texturing a texturing-use material containing the enzyme-treated plant protein material. The following describes in detail the method for producing a textured plant protein according to the present invention.

1-1. Step 1

In step 1, an untextured plant protein material is treated with a glutaminase compound and a protease to prepare an enzyme-treated plant protein material.

1-1-1. Untextured plant protein material

An untextured plant protein material (hereinafter, also simply referred to as "plant protein material") refers to an untextured material obtained by using a plant as a raw material, and increasing the ratio of the content of protein contained in the plant per dry weight by performing a treatment such as removing at least some components other than protein among constituent components of the plant.

The plant is not particularly limited, and examples include pulses (e.g., a soybean, pea, lentil, chickpea, black bean, bean, mung bean, mung bean, lupin bean, kidney bean); cereal crops (e.g., wheat, barley, oats, sorghum, rice, rye, buckwheat, barnyard millet, foxtail millet, teff, quinoa, corn, potato); seeds (e.g., an almond, coconut, peanut, cashew nut, hazelnut, pecan nut, macadamia nut, pistachio, walnut, Brazil nut, pili nut, chestnut, sesame, pine nut, hemp seed (industrial hemp), chia seed, chia, amaranth, canary seed, flaxseed); or algae.

The plant protein material may be obtained from one of these plants or may be obtained from a plurality of plants. Among these plants, pulses are preferable, a soybean is more preferable, and a pea is still more preferable.

The content of the plant protein in the plant protein material (dry weight) is not particularly limited, and is, for example, 40 wt% or more, preferably 50 wt% or more, more preferably 60 wt% or more, still more preferably 70 wt% or more, and still more preferably 75 wt% or more. The content of the plant protein in the plant protein material (dry weight) is not particularly limited even at the upper limit, and is, for example, 98 wt% or less, preferably 95 wt% or less, more preferably 90 wt% or less, and still more preferably 85 wt% or less.

1-1-2. Glutaminase compound (glutaminases)

In the present invention, "glutaminase compound (glutaminases)" is a generic term for enzymes having an activity of hydrolyzing a γ-glutamyl bond, and is not particularly limited as long as the enzymes have the activity. Thus, the glutaminases may be either those having free glutamine as a substrate or those having a glutamine residue as a substrate, and may be either those without transfer activity or those with transfer activity. Examples of the glutaminase compound (glutaminases) that can be used in the present invention include a glutaminase (EC3.5.1.2; glutaminase; enzyme that hydrolyzes free glutamine to glutamic acid), a protein glutaminase (EC3.5.1.XX; protein glutaminase; enzyme that deamidate and convert glutamine residues in proteins or peptides into glutamic acid residues), and a transglutaminase (EC2.3.2.13; transglutaminase; enzyme that transfers a glutamine residue in a protein or peptide to another amino acid residue to form an iso-peptide bridge). In the present invention, as the glutaminase, one of these enzymes may be used singly, or two or more of these enzymes may be used in combination. Among these glutaminases, a glutaminase (EC3.5.1.2; glutaminase; enzyme that hydrolyzes free glutamine to glutamic acid) is preferable from the viewpoint of further enhancing the effect of suppressing color dullness of the textured plant protein.

The glutaminases are not particularly limited in terms of their origin, and specific examples thereof include glutaminases derived from microorganisms such as the genus Bacillus (preferably, glutaminase (EC3.5.1.2) derived from microorganisms such as the genus Bacillus). From the viewpoint of further enhancing the effect of suppressing color dullness of the textured plant protein, preferred examples thereof include glutaminases derived from the genus Bacillus (preferably glutaminase (EC3.5.1.2) derived from the genus Bacillus), and more preferred examples thereof include glutaminases derived from Bacillus amyloliquefaciens (preferably glutaminase (EC3.5.1.2) derived from Bacillus amyloliquefaciens).

When a microorganism-derived glutaminase compound is used, a culture medium of a microorganism from which the glutaminase compound is derived, a lysate thereof, and an extract thereof may be used, or a purified product obtained by purifying them at a given level and thus increasing the content of the glutaminase compound may be used. In addition, when a glutaminase compound is used, a commercially available enzyme preparation (e.g., glutaminase derived from Bacillus amyloliquefaciens, as manufactured by Amano Enzyme Inc.) may be used.

The amount of glutaminase compound used is not particularly limited, and the amount used per g of plant protein is, for example, 0.001 U or more. From the viewpoint of further enhancing the effect of suppressing color dullness of the textured plant protein, the amount of glutaminase compound used per g of protein is preferably 0.005 U or more, more preferably 0.01 U or more, 0.05 U or more, 0.1 U or more, 0.3 U or more, 0.4 U or more, 0.5 U or more, or 0.6 U or more, still more preferably 0.7 U or more, still more preferably 0.8 U or more, and still more preferably 0.9 U or more. The amount of glutaminase compound used is not particularly limited even at the upper limit thereof, and the amount used per g of plant protein is, for example, 100 U or less, or 50 U or less, preferably 10 U or less, and more preferably 8 U or less, 6 U or less, 4 U or less, 3 U or less, 2 U or less, or 1.5 U or less.

The amount of glutaminase compound used per g of the plant protein material is, for example, 0.0008 U or more. From the viewpoint of further enhancing the effect of suppressing color dullness of the textured plant protein, the amount of glutaminase compound used per g of protein material is preferably 0.004 U or more, more preferably 0.008 U or more, 0.04 U or more, 0.08 U or more, 0.24 U or more, 0.32 U or more, 0.4 U or more, or 0.48 U or more, still more preferably 0.56 U or more, still more preferably 0.64 U or more, and still more preferably 0.72 U or more. The amount of glutaminase compound used is not particularly limited even at the upper limit thereof, and the amount used per g of plant protein material is, for example, 80 U or less, or 40 U or less, preferably 8 U or less, and more preferably 6.4 U or less, 4.8 U or less, 3.2 U or less, 2.4 U or less, 1.6 U or less, or 1.2 U or less.

Note that the activity of glutaminase compound is defined such that the enzyme amount that produces 1 $\mu$mol of L-glutamic acid per minute while using L-glutamine as a substrate is 1 unit (1 U).

1-1-3. Protease

The protease is not particularly limited as long as it is an endopeptidase. Examples of the protease include proteases derived from filamentous fungi and proteases derived from bacteria. As the protease, either one of a filamentous fungus-derived protease and a bacterium-derived protease may be used, or both may be used in combination. From the viewpoint of further enhancing the effect of suppressing color dullness of the textured plant protein, it is preferable to use a combination of both a filamentous fungus-derived protease and a bacterium-derived protease.

The filamentous fungus-derived protease is not particularly limited, and examples thereof include proteases derived from the genus Aspergillus, the genus Rhizopus, the genus Mucor, the genus Neurospora, the genus Penicillium, the genus Rhizomucor, the genus Sclerotinia, and others. Examples of the protease derived from the genus Aspergillus include a protease derived from Aspergillus oryzae or a protease derived from Aspergillus melleus. The filamentous fungus-derived protease may be an acidic protease or a neutral protease. More specific examples of the filamentous fungus-derived protease include an acidic protease derived from Aspergillus oryzae, a neutral protease derived from Aspergillus oryzae, or a neutral protease derived from Aspergillus melleus.

One kind of these filamentous fungus-derived proteases may be used singly, or multiple kinds thereof may be used in combination. Among these proteases derived from filamentous fungi, a protease derived from the genus Aspergillus is preferable, a protease derived from Aspergillus oryzae is more preferable, and an acidic protease derived from Aspergillus oryzae is still more preferable, from the viewpoint of further enhancing the effect of suppressing color dullness of the textured plant protein.

When a filamentous fungus-derived protease is used, a culture medium of each filamentous fungus described above, a lysate thereof, and an extract thereof may be used, or a purified product obtained by purifying them at a given level and thus increasing the content of the protease may be used. In addition, when a filamentous fungus-derived protease is used, a commercially available enzyme preparation (e.g., an acidic protease derived from Aspergillus oryzae, a neutral protease derived from Aspergillus oryzae, and/or a neutral protease derived from Aspergillus melleus, as manufactured by Amano Enzyme Inc.) may be used.

The bacterial protease is not particularly limited, and examples thereof include proteases derived from the genus Bacillus, the genus Geobacillus, and others.

Specific examples of the protease derived from the genus Bacillus (or the genus Geobacillus) include proteases derived from Bacillus amyloliquefaciens, Bacillus cereus, Bacillus clausii, Bacillus intermedius, Bacillus lentus, Bacillus licheniformis, Bacillus stearothermophilus, Bacillus subtilis, and Bacillus thermoproteolyticus, and proteases derived from the genus Geobacillus.

One kind of these proteases derived from the genus Bacillus (or the genus Geobacillus) may be used singly, or multiple kinds thereof may be used in combination. Among these proteases derived from the genus Bacillus (or the genus Geobacillus), Bacillus stearothermophilus or Geobacillus stearothermophilus is preferable from the viewpoint of further enhancing the effect of suppressing color dullness of the textured plant protein.

When a bacterium-derived protease is used, a culture medium of each bacterium described above, a lysate thereof, and an extract thereof may be used, or a purified product obtained by purifying them at a given level and thus increasing the content of the protease may be used. In addition, when a bacterium-derived protease is used, a commercially available enzyme preparation (e.g., protease derived from Geobacillus stearothermophilus, as manufactured by Amano Enzyme Inc.) may be used.

The amount (total amount) of protease(s) used is not particularly limited, and the amount used per g of plant protein is, for example, 4 U or more. From the viewpoint of further enhancing the effect of suppressing color dullness of the textured plant protein, the amount of protease used per g of protein is preferably 40 U or more, more preferably 400 U or more, still more preferably 1,000 U or more, still more preferably 2,000 U or more, and still more preferably 3,000 U or more. The amount (total amount) of protease(s) used is not particularly limited even at the upper limit thereof, and the amount used per g of plant protein is, for example, 100,000 U or less or 50,000 U or less, preferably 10,000 U or less, more preferably 8,000 U or less or 6,000 U or less.

In addition, the amount (total amount) of protease(s) used per g of the plant protein material is, for example, 3.2 U or more. From the viewpoint of further enhancing the effect of suppressing color dullness of the textured plant protein, the amount of protease used per g of protein material is preferably 32 U or more, more preferably 320 U or more, still more preferably 800 U or more, still more preferably 1,600 U or more, and still more preferably 2,400 U or more. The amount (total amount) of protease(s) used is not particularly limited even at the upper limit thereof, but the amount used per g of plant protein material is, for example, 80,000 U or less, or 40,000 U or less, preferably 8,000 U or less, and more preferably 6,400 U or less or 4,800 U or less.

When both the filamentous fungus-derived protease and the bacterium-derived protease are used in combination, the ratio is not particularly limited. The amount of bacterium-derived protease used per U of filamentous fungus-derived protease is, for example, from 0.01 to 2 U, preferably from 0.05 to 1 U, more preferably from 0.1 to 0.7 U, still more preferably from 0.2 to 0.5 U, and still more preferably from 0.3 to 0.4 U.

The protease activity is defined such that the enzyme amount that produces an increase in the amount of the Folin test solution coloring substance corresponding to 1 $\mu$g of tyrosine per minute while casein is used as a substrate is 1 unit (1 U).

1-1-4. Enzymatic treatment reaction

The enzymatic treatment reaction in which a glutaminase compound and a protease act on an untextured plant protein material can be usually performed by subjecting an enzymatic treatment-use mixed solution containing an untextured plant protein material, a glutaminase compound, and a protease in water to reaction conditions of the enzymes.

The dry weight of the plant protein material in the enzymatic treatment-use mixed solution is not particularly limited, and is, for example, 1 to 30 wt%, preferably 3 to 20 wt%, more preferably 5 to 15 wt%, and still more preferably 8 to 12 wt%.

The enzymatic treatment with a glutaminase compound and the enzymatic treatment with a protease may be performed simultaneously or stepwise. When these enzymatic treatments are performed simultaneously, an enzymatic treatment-use mixed solution containing an untextured plant protein material, a glutaminase compound, and a protease in water is prepared, and the mixed solution is then subjected to reaction conditions of the enzymes. When these enzymatic treatments are performed stepwise, a first enzymatic treatment-use mixed solution containing an untextured plant protein material and either one of a glutaminase compound or a protease in water is prepared; the first mixed solution is then subjected to reaction conditions of the one enzyme; the first enzymatic treatment-use mixed solution is then admixed with the other of the glutaminase compound or the protease to prepare a second enzymatic treatment-use mixed solution; and the second mixed solution is then subjected to reaction conditions of the other enzyme.

The reaction conditions (temperature and pH) may be set, if appropriate, according to the thermal characteristics and pH characteristics of the enzyme used. Specific examples of the reaction temperature include 15°C to 70°C, preferably 30°C to 65°C, and more preferably 40°C to 60°C. Specific examples of the pH (at 25°C) of the enzymatic treatment-use mixed solution include pH 3 to 11, preferably pH 4 to 10, more preferably pH 5 to 9, and still more preferably pH 6 to 8. In addition, the reaction time is, for example, minutes to 48 hours, preferably 10 minutes to 24 hours, and more preferably 15 minutes to 12 hours.

1-1-5. Enzyme-treated plant protein material (enzyme-treated product)

The form of the enzyme-treated plant protein material (hereinafter, also called "enzyme-treated product") obtained in step 1 is not particularly limited as long as the form is applicable to step 2. Specific examples of the form of the enzyme-treated product include a liquid form, a paste form, and a solid form (e.g., in the form of powder, granules), and the form is preferably a solid form, and more preferably a powder form.

The enzyme-treated product may be blended in a texturing-use material to suppress color dullness of the resulting textured plant protein.

1-2. Step 2

In step 2, a texturing-use material containing the enzyme-treated plant protein material (enzyme-treated product) obtained in step 1 is subjected to texturing treatment.

1-2-1. Texturing-use material

The composition of the texturing-use material is not particularly limited as long as it contains the enzyme-treated plant protein material (enzyme-treated product) obtained in step 1 and is prepared as a kneaded material allowing for texturing.

Examples of components, other than the enzyme-treated product, that can be blended in the texturing-use material include a plant protein material (untextured material that has not undergone the enzymatic treatment in step 1), polysaccharides, water, and oil.

As a specific example of the plant protein material that can be blended in the texturing-use material, one or more kinds can be selected from specific examples of the untextured plant protein material in step 1. Examples include a plant protein material derived from preferably pulses, more preferably a soybean or a pea, and still more preferably a pea. As the polysaccharide, one or more kinds can be selected from dextrin, cellulose, starch, dietary fiber, and so on, and dextrin is preferable. The oil is preferably a plant oil.

The content (dry weight) of the enzyme-treated product based on 100 parts by weight (dry weight) of the texturing-use material can be set, if appropriate, according to the desired degree of the effect of suppressing color dullness of the textured plant protein. Specific examples of the content (dry weight) of the enzyme-treated product based on 100 parts by weight (dry weight) of the texturing-use material include 3 parts by weight or more. From the viewpoint of further enhancing the effect of suppressing color dullness of the textured plant protein, the content (dry weight) is preferably 5 parts by weight or more, more preferably 10 parts by weight or more, still more preferably 13 parts by weight or more, and still more preferably 15 parts by weight or more. The content (dry weight) of the enzyme-treated product based on 100 parts by weight (dry weight) of the texturing-use material is not particularly limited even at the upper limit, and is, for example, 100 parts by weight or less, 80 parts by weight or less, 60 parts by weight or less, 40 parts by weight or less, or 30 parts by weight or less.

When the texturing-use material contains a plant protein material, the content (dry weight) of the plant protein material based on 100 parts by weight (dry weight) of the texturing-use material is not particularly limited, but is, for example, 40 parts by weight or more, 50 parts by weight or more, 60 parts by weight or more, 65 parts by weight or more, 70 parts by weight or more, or 75 parts by weight or more. The content (dry weight) of the plant protein material based on 100 parts by weight (dry weight) of the texturing-use material is not particularly limited even at the upper limit, and is, for example, 90 parts by weight or less, preferably 85 parts by weight or less, more preferably 80 parts by weight or less, and still more preferably 75 parts by weight or less.

When the texturing-use material contains a plant protein material, the content (dry weight) of the enzyme-treated product based on 100 parts by weight (dry weight) of the plant protein material is, for example, 5 parts by weight or more. From the viewpoint of further enhancing the effect of suppressing color dullness of the textured plant protein, the content (dry weight) is preferably 10 parts by weight or more, more preferably 15 parts by weight or more, still more preferably 18 parts by weight or more, and still more preferably 20 parts by weight or more. The content (dry weight) of the enzyme-treated product based on 100 parts by weight (dry weight) of the plant protein material is not particularly limited even at the upper limit, and is, for example, 80 parts by weight or less, 60 parts by weight or less, 40 parts by weight or less, 30 parts by weight or less, or 25 parts by weight or less.

When the texturing-use material contains a plant protein material, the total amount (dry weight) of the plant protein material and enzyme-treated product based on 100 parts by weight (dry weight) of the texturing-use material is, for example, 50 parts by weight or more, preferably 60 parts by weight or more, more preferably 70 parts by weight or more, still more preferably 80 parts by weight or more, and still more preferably 83 parts by weight or more. The total amount (dry weight) of the plant protein material and enzyme-treated product based on 100 parts by weight (dry weight) of the texturing-use material is not particularly limited even at the upper limit, and is, for example, 95 parts by weight or less, 90 parts by weight or less, or 87 parts by weight or less.

When the texturing-use material contains a polysaccharide, the content (dry weight) of the polysaccharide based on 100 parts by weight (dry weight) of the texturing-use material is not particularly limited, but it is, for example, 5 parts by weight or more, preferably 10 parts by weight or more, and more preferably 13 parts by weight or more. The content (dry weight) of the polysaccharide based on 100 parts by weight (dry weight) of the texturing-use material is not particularly limited even at the upper limit, and is, for example, 30 parts by weight or less, preferably 25 parts by weight or less, more preferably 20 parts by weight or less, and still more preferably 18 parts by weight or less.

1-2-2. Texturing treatment

The specific technique for the texturing treatment is not particularly limited as long as it is a method for preparing a textured plant protein, and specific examples thereof include extrusion at high temperature and high pressure by using an extruder. The extruded material can also be further subjected to drying and/or freezing, and preferably lyophilization.

1-2-3. Textured plant protein

The textured plant protein obtained by the present invention can be generally used as substitute meat (pseudo meat). The "meat" imitated by the textured plant protein obtained by the present invention means muscles of an edible animal, and when described as "meat", the "meat" is used in the sense of including not only muscles of mammals and birds but also fish and shellfish.

Examples of the shape of the textured plant protein include a granular shape and a fibrous shape. Examples of the granular shape include lump shapes with various sizes such as a small grain type (mince), a large grain type, and a block type (the size increases in the order of the small grain type, the large grain type, and the block type); or flat shapes with various sizes such as a flake type, a fillet type, and a slice type (the size increases in the order of the flake type, the fillet type, and the slice type).

2. Food containing textured plant protein

The present invention also provides a food containing the textured plant protein obtained by the above "1. Method for producing textured plant protein".

A specific form of the food of the present invention may be selected from food forms, and can conform to foods such as processed livestock meat, chicken meat, and/or fish meat. That is, examples of the food of the present invention include meat-like processed foods (refer to foods imitating processed livestock meat, chicken meat, and/or fish meat foods). More preferred examples thereof include processed meat and/or chicken foods (refer to foods imitating processed meat and/or chicken foods). Such a processed livestock meat and/or chicken meat food may be a food which is cooked by molding and heating a sort of meat using the livestock meat and/or the chicken meat. Specific examples thereof include Hamburg steak, meat ball, patty, meat loaf, minced cutlet, or dim sum.

The food of the present invention may be obtained by subjecting the textured plant protein obtained by the above production method to an optional preparing process. In the preparing process, for instance, swelling, coloring, seasoning, form adjustment, molding, heating cooking, fermentation, and/or freezing may be performed. Examples of a specific method in the preparing process include a method of swelling with water as necessary, and/or adding a coloring agent and other additives and/or seasoning to mold the mixture into a desired shape, and further heating and cooking the mixture as necessary. A specific method of heating and cooking can be appropriately determined by those skilled in the art according to the type of food, and specific examples include boiling, firing (e.g., roasting, toasting, baking, grilling, broiling), steaming, and/or frying.

3. Color dullness inhibitor for textured plant protein

As described above, the enzyme-treated plant protein material (enzyme-treated product) obtained in step 1 of the above "1. Method for producing textured plant protein" may be blended in a texturing-use material to suppress color dullness of the resulting textured plant protein. Thus, the present invention also provides a color dullness inhibitor for a textured plant protein, including a product obtained by enzymatic treatment of an untextured plant protein material with a glutaminase compound and a protease.

The suppression of color dullness of the textured plant protein means an increase in the brightness and yellowness of the grayish brown color originally exhibited by the appearance of the textured plant protein (those produced without using the enzyme-treated product obtained in step 1 of the above "1. Method for producing textured plant protein"). Specifically, the suppression can be checked by whether the L* value and the b* value in accordance with JIS (JIS Z 8781-4) are increased.

In the color dullness inhibitor for a textured plant protein, components to be used, amounts to be used, specific methods to be used, and others are as indicated in the Section "1. Method for producing textured plant protein" above.

4. Coloring inhibitor for textured plant protein

The grayish brown color exhibited by the textured plant protein is considered to be a result of coloring by a brown substance generated by a Maillard reaction between sugars and amino acids contained in the plant material at least in any process until the plant material is formed into a textured form. Here, the enzyme-treated plant protein material (enzyme-treated product) obtained in step 1 of the above "1. Method for producing textured plant protein" may be blended in a texturing-use material to suppress color dullness of the resulting textured plant protein. It is considered that the combination of a glutaminase compound and a protease used in the enzymatic treatment suppresses the Maillard reaction between sugars and amino acids contained in the plant material, thereby inhibiting the coloring of the textured plant protein. Therefore, the present invention also provides a coloring inhibitor for a textured plant protein, including a glutaminase compound and a protease.

In the coloring inhibitor for a textured plant protein, components to be used, amounts to be used, specific methods to be used, and others are as indicated in the Section "1. Method for producing textured plant protein" above.

Examples

Hereinafter, the present invention will be described in more detail with reference to Examples below. The present invention, however, is not limited to these Examples.

(1) Materials

(1-1) Untextured plant protein material

· Pea proteins: PURIS Pea870 (manufactured by PURIS; protein content: 80 wt%)

(1-2) Enzyme

· Glutaminase compound: Glutaminase (EC3.5.1.2) derived from Bacillus amyloliquefaciens (Amano Enzyme Inc.)
· Filamentous fungus-derived protease: Aspergillus oryzae-derived acidic protease

(Amano Enzyme Inc.)

· Bacterium-derived protease: Geobacillus stearothermophilus-derived protease (Amano Enzyme Inc.)

(2) How to measure enzyme activity value

(2-1) Protease activity assay

First, 5 mL of 0.6% (w/v) casein solution (0.05 mol/L sodium hydrogen phosphate, at pH 8.0 [in the case of bacterium-derived protease]) or 0.6% (w/v) casein solution (0.7% (w/v) lactic acid, at pH 3.0 [in the case of filamentous fungus-derived protease]) was heated at 37°C for 10 minutes, 1 mL of a sample solution containing a protease was then added, and the mixture was immediately shaken. The resulting solution was allowed to stand at 37°C for 10 minutes, 5 mL of trichloroacetic acid test solution (aqueous solution containing 1.8% (w/v) trichloroacetic acid, 1.8% (w/v) sodium acetate, and 0.33 mol/L acetic acid) was then added, and the mixture was shaken, allowed to stand again at 37°C for 30 minutes, and filtered. The first filtrate (3 mL) was removed, and the next filtrate (2 mL) was weighed. Then, 5 mL of 0.55 mol/L sodium carbonate test solution and 1 mL of Folin test solution (1→3) were added, and the mixture was shaken well and left at 37°C for 30 minutes. With respect to this liquid (enzyme reaction liquid), absorbance AT at a wavelength of 660 nm was measured using water as a control.
Separately, 1 mL of the sample solution containing a protease was weighed, and 5 mL of trichloroacetic acid test solution was added and shaken, then 5 mL of casein solution at a measurement pH set for each sample was added, and the mixture was immediately shaken and left at 37°C for 30 minutes. Except for that, the absorbance AB of a liquid (blank) that was manipulated in the same manner as for the above-described enzyme reaction liquid was measured.
The enzyme amount that produced an increase in the amount of the Folin test solution coloring substance corresponding to 1 μg of tyrosine per minute was defined as 1 unit (1 U).
Next, 1 mL, 2 mL, 3 mL, and 4 mL of 1 mg/mL tyrosine standard stock solution (0.2 mol/L hydrochloric acid) were each weighed, and 0.2 mol/L hydrochloric acid test solution was added thereto to make 100 mL. Then, 2 mL of each liquid was weighed and 5 mL of 0.55 mol/L sodium carbonate test solution and 1 mL of Folin test solution (1→3) were added, and the mixture was shaken immediately and left at 37°C for 30 minutes. For these liquids, absorbances A1, A2, A3 and A4 at a wavelength of 660 nm were measured using, as a control, a liquid obtained by weighing 2 mL of 0.2 mol/L hydrochloric acid reagent and manipulating in the same manner as described above. The absorbances A1, A2, A3 and A4 were plotted on the vertical axis, and the amount of tyrosine (μg) in 2 mL of each liquid was plotted on the horizontal axis. A calibration curve was thus prepared to determine the amount of tyrosine (μg) corresponding to the absorbance difference 1.

[Mathematical Formula 1]

$$\text{Protease activity (U/g, U/mL)} = (AT - AB) \times F \times 11/2 \times 1/10 \times 1/M$$

AT: Absorbance of enzyme reaction liquid
AB: Absorbance of blank
F: Amount (μg) of tyrosine when the absorbance difference determined from a tyrosine calibration curve is 1

11/2: Coefficient of conversion to a total liquid volume after a reaction is stopped

1/10: Conversion coefficient per minute of reaction time

M: Amount (g or mL) of sample in 1 mL of sample solution

(2-2) Glutaminase compound activity assay

First, 1 mL of enzyme solution was weighed in a test tube and left in a constant temperature water bath at 37°C for 5 minutes. This was admixed with 1 mL of 2% (w/v) L-glutamine solution (0.1 mol/L acetate buffer (pH 6.0)) preheated to 37°C, and the mixture was allowed to stand for exactly 10 minutes. After standing, 1 mL of 5% (v/v) perchloric acid test solution was admixed, and the mixture was immediately placed in ice-water. After leaving for 1 minute or more, 1 mL of sodium hydroxide test solution (0.75 mol/L) was admixed to prepare a reaction liquid. L-Glutamic acid in the reaction liquid was quantified using an L-glutamic acid assay kit "YAMASA" NEO (manufactured by YAMASA SHOYU CO., LTD.). Under these conditions, the enzyme amount that produced 1 μmol of L-glutamic acid per minute was defined as 1 unit.

Test Examples

1. To prepare enzyme-treated plant protein material (enzyme-treated product powder)

Pea proteins were suspended in water to about 10 wt% and the temperature was adjusted to 50°C with stirring. Then, enzymes (glutaminase derived from Bacillus amyloliquefaciens: 1U/g protein; acidic protease derived from Aspergillus oryzae: 3,000U/g protein; and protease derived from Geobacillus stearothermophilus: 1,000U/g protein) were added to the pea protein raw material, and the mixture was reacted at 50°C for 1 hour. Thereafter, the temperature was raised to 85°C and then held for 10 minutes to deactivate the enzymes. The resulting enzyme-treated protein solution was powdered with a spray dryer (Model: Henningsen Pilot Plant Tower Spray Dryer Model T-20; inlet temperature: 140 to 150°C; outlet temperature: 90 to 100°C) to prepare an enzyme-treated plant protein material (enzyme-treated product powder).

2. To prepare textured plant protein

An appropriate amount of water was added to each composition shown in the following Table while using the enzyme-treated product powder, and the mixture was kneaded and textured (pressure: 150-175 psi; stage 1 temperature: 60°C, stage 2 temperature: 80°C, stage 3 temperature: 90°C, stage 4 temperature: 100°C) with an extruder (Wenger Manufacturing TX-57 model) to prepare a textured protein (granular).

[Table 1]

|  | Composition |
|---|---|
| Comparative Example 1 | 15 wt% Dextrin (Pinedex #2, Matsutani Chemical Industry Co., Ltd.) 85 wt% Pea proteins (PURIS Pea870, PURIS) |
| Example 1 | 15 wt% Dextrin (Pinedex #2, Matsutani Chemical Industry Co., Ltd.) 85 wt% Pea proteins (PURIS Pea870, PURIS) 5% Enzyme-treated product powder |
| Example 2 | 15 wt% Dextrin (Pinedex #2, Matsutani Chemical Industry Co., Ltd.) 70 wt% Pea proteins (PURIS Pea870, PURIS) 5% Enzyme-treated product powder |

3. To evaluate color tone of textured plant protein

The L*a*b* value of each textured plant protein (dry state) obtained by the above method was measured using a spectrocolorimeter (CM-700d: Konica Minolta Sensing Co., Ltd.), and the color tone was quantified as a number. The L* value represents brightness. As the L* value becomes larger, the color is closer to white, and as the L* value becomes smaller, the color is closer to black. The a* value represents chromaticity. As the a* value becomes larger, the redness becomes stronger, and as the a* value becomes smaller, the greenness becomes stronger. The b* value represents chromaticity. As the b* value becomes larger, the yellowness becomes stronger, and as the b* value becomes smaller, the blueness becomes stronger. Table 2 shows the results. Fig. 1 is a photograph showing the appearance of each textured plant protein (dry state) obtained by the above method.

[Table 2]

|  | L* value | a* value | b* value |
|---|---|---|---|
| Comparative Example 1 | 63.2 | 6.4 | 19.6 |
| Example 1 | 65.2 | 6.2 | 20.8 |
| Example 2 | 67.8 | 6.3 | 22.1 |

As shown in Table 2, the textured plant proteins of Examples 1 and 2 as produced by blending the enzyme-treated product powder in a texturing-use material had a higher L* value and b* value than the textured plant protein of Comparative Example 1 as produced without using the enzyme-treated protein powder. It was found that this trend increased as the blending ratio of the enzyme-treated protein powder became larger. That is, the textured plant protein of Comparative Example 1 as produced without using the enzyme-treated protein powder was grayish brown. By contrast, the textured plant proteins of Examples 1 and 2 as produced by blending the enzyme-treated product powder in a texturing-use material had increased brightness and yellowness, thereby suppressing dullness of the grayish brown color. The increase in the brightness and yellowness therefore suppressed color dullness of the textured plant protein. This was also visually confirmed from the appearance of each textured plant protein as shown in Fig. 1.

## Claims

1. A method for producing a textured plant protein, comprising:

   step 1 of treating an untextured plant protein material with a glutaminase compound and a protease to prepare an enzyme-treated plant protein material; and
   step 2 of texturing a texturing-use material containing the enzyme-treated plant protein material.

2. The method according to claim 1, wherein a dry weight of the enzyme-treated plant protein material is 3 parts by weight or more based on 100 parts by weight of a dry weight of the texturing-use material.

3. The method according to claim 1, wherein the glutaminase compound is a glutaminase.

4. The method according to claim 1, wherein the glutaminase compound is a glutaminase compound derived from the genus Bacillus.

5. The method according to claim 1, wherein the protease is a filamentous fungus-derived protease and/or a bacterium-derived protease.

6. The method according to claim 5, wherein the filamentous fungus protease is an acidic protease.

7. The method according to claim 5, wherein the filamentous fungus protease is a protease derived from the genus Aspergillus.

8. The method according to claim 5, wherein the bacterium protease is a protease derived from the genus Bacillus or the genus Geobacillus.

9. The method according to claim 1, wherein the plant protein material is derived from pulses.

10. The method according to claim 1, wherein the protein material is derived from a pea.

11. A food comprising a textured plant protein produced by the method according to claim 1.

12. A color dullness inhibitor for a textured plant protein, comprising a product obtained by enzymatic treatment of an untextured plant protein material with a glutaminase compound and a protease.

13. A coloring inhibitor for a textured plant protein, comprising a glutaminase compound and a protease.

FIG. 1

Comparative Example 1 Example 1 Example 2

## INTERNATIONAL SEARCH REPORT

International application No.

**PCT/JP2023/039955**

### A. CLASSIFICATION OF SUBJECT MATTER

*A23J 3/14*(2006.01)i; *A23J 3/22*(2006.01)i; *A23J 3/34*(2006.01)i; *A23L 5/00*(2016.01)i; *A23L 11/00*(2021.01)i; *A23L 13/00*(2016.01)i; *C12N 9/48*(2006.01)i; *C12N 9/54*(2006.01)i; *C12N 9/62*(2006.01)i; *C12P 1/00*(2006.01)i; *C12P 21/06*(2006.01)i

FI:  A23J3/14; A23J3/22; A23J3/34; A23L5/00 J; A23L11/00 F; A23L13/00 A; A23L13/00 D; A23L13/00 Z; C12N9/48; C12N9/54; C12N9/62; C12P1/00 A; C12P21/06

According to International Patent Classification (IPC) or to both national classification and IPC

### B. FIELDS SEARCHED

Minimum documentation searched (classification system followed by classification symbols)

A23J3/14; A23J3/22; A23J3/34; A23L5/00; A23L11/00; A23L13/00; C12N9/48; C12N9/54; C12N9/62; C12P1/00; C12P21/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2006-141231 A (FUJI OIL CO., LTD.) 08 June 2006 (2006-06-08)<br>claims | 1-13 |
| A | WO 2022/097745 A1 (AMANO ENZYME INC.) 12 May 2022 (2022-05-12)<br>claims | 1-13 |
| A | KR 10-2020-0012170 A (KOREA FOOD RESEARCH INSTITUTE) 05 February 2020 (2020-02-05)<br>claims | 1-13 |
| A | WO 2022/215688 A1 (AMANO ENZYME EUROPE LTD.) 13 October 2022 (2022-10-13)<br>claims | 1-13 |

☑ Further documents are listed in the continuation of Box C.   ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **12 January 2024** | **23 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

International application No.

**PCT/JP2023/039955**

### C.   DOCUMENTS CONSIDERED TO BE RELEVANT

| Category* | Citation of document, with indication, where appropriate, of the relevant passages | Relevant to claim No. |
|---|---|---|
| A | WO 2022/191303 A1 (AMANO ENZYME U.S.A. CO., LTD.) 15 September 2022 (2022-09-15)<br>claims | 1-13 |
| A | WO 2022/102723 A1 (AMANO ENZYME INC.) 19 May 2022 (2022-05-19)<br>claims | 1-13 |

Form PCT/ISA/210 (second sheet) (July 2022)

### INTERNATIONAL SEARCH REPORT
**Information on patent family members**

International application No.

**PCT/JP2023/039955**

| Patent document cited in search report | | | Publication date (day/month/year) | Patent family member(s) | | | Publication date (day/month/year) |
|---|---|---|---|---|---|---|---|
| JP | 2006-141231 | A | 08 June 2006 | (Family: none) | | | |
| WO | 2022/097745 | A1 | 12 May 2022 | EP | 4241570 | A1 | |
| | | | | claims | | | |
| | | | | CN | 116322347 | A | |
| KR | 10-2020-0012170 | A | 05 February 2020 | (Family: none) | | | |
| WO | 2022/215688 | A1 | 13 October 2022 | CN | 116916759 | A | |
| | | | | claims | | | |
| WO | 2022/191303 | A1 | 15 September 2022 | (Family: none) | | | |
| WO | 2022/102723 | A1 | 19 May 2022 | EP | 4245149 | A1 | |
| | | | | claims | | | |
| | | | | CN | 116471944 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 1491572 A

**Non-patent literature cited in the description**

- YES, IT'S MEAT MADE FROM PLANTS. *BEYOND MEAT*, 07 November 2022, https://www.beyond-meat.com/en-US/about/our-ingredients/>

- *International Journal of Food Properties*, 2019, vol. 22, 1283-1295